# NEW EUROPEAN PATENT SPECIFICATION

(11) **EP 1 349 497 B2**
(45) Date of publication and mention of the opposition decision: **21.09.2011**
(45) Mention of the grant of the patent: 25.07.2007
(21) Application number: 02758541.3
(22) Date of filing: 16.08.2002
(51) Int. Cl.: A61B 5/15, A61B 5/00

(54) **IN-SITU ADAPTER FOR AN ANALYTE TESTING DEVICE**
IN-SITU ADAPTER FÜR EIN ANALYTEN-TESTGERÄT
ADAPTATEUR IN-SITU POUR DISPOSITIF D'ESSAI

(30) Priority: 16.08.2001 US 313059 P
(43) Date of publication of application: 08.10.2003
(60) Divisional application: 07014058.7
(73) Proprietor: Lifescan Scotland Ltd, Inverness, IV2 3ED, Scotland (GB)
(72) Inventor: MOERMAN, Piet, B-9831 St. Martens-Latem (BE); GRIFFITH, Alun, Inverness IV2 3XQ (GB)
(74) Representative: Mercer, Christopher Paul
(86) International application number: PCT/GB2002/003772
(87) International publication number: WO 2003/015627

(56) References cited:
- WO-A-01/28423
- WO-A-97/42882
- WO-A-99/27854
- WO-A1-99/22236
- DE-A- 4 234 553
- DE-A1- 19 819 407
- US-A- 4 895 147
- US-A- 4 959 196
- US-A- 5 231 993
- US-A- 5 514 152
- US-A- 5 772 586
- US-A- 5 871 494
- US-A- 6 036 924

## Description

### FIELD OF THE INVENTION

The present invention relates to an adapter for a testing device for sampling and analyzing analytes in bodily fluids, in particular a glucose meter for sampling and analyzing glucose in blood or interstitial fluid.

### BACKGROUND OF THE INVENTION

Glucose monitoring is a fact of everyday life for diabetic individuals. The accuracy of such monitoring can significantly affect the health and ultimately the quality of life of a person with diabetes. Generally, a diabetic patient measures blood glucose levels several times a day to monitor and control blood sugar levels. Failure to test blood glucose levels accurately and on a regular basis can result in serious diabetes-related complications, including cardiovascular disease, kidney disease, nerve damage and blindness. A number of glucose meters are currently available which permit an individual to test the glucose level in a small sample of blood.

Many of the glucose meter designs currently available make use of a disposable test strip, which, in combination with the meter, electrochemically measures the amount of glucose in the blood sample. To use these meters, the user first pricks a finger or other body part using a separate lancing device to produce a small sample of blood or interstitial fluid. The sample is then transferred to a disposable test strip having an electrode system. The test strip is then inserted into the glucose meter to measure the glucose level of the sample. The test strip may be inserted into the meter prior to obtaining a blood sample. The inconvenience of taking several measurements a day as well as the pain inflicted by currently available lancets or finger-sticking devices often discourage disciplined and frequent testing.

While the fingertip is generally used for sampling blood, due to the rich capillary bed of the skin of the fingertip, the fingertip is also particularly sensitive to pain, due to a rich supply of pain receptors in the fingertip as well. When the incision from a lancet is too deep, too close to a recent incision or not deep enough and requires an additional incision, the pain from a puncture is increased significantly. Pain may also be increased if the cutting blade penetrates slowly or is withdrawn slowly. Furthermore, the user may be forced to make a larger incision than is necessary to form a sufficient amount of blood, due to losses incurred during the transfer of the sample between the puncture site and the test strip.

The process of monitoring blood glucose levels requires several steps and several different accessories, including a lancing device, a lancet, a supply of test strips and a glucose meter. Each accessory has a different function. The user must have a flat surface available to unpack and lay down the accessories within easy reach. First, the user charges the lancing device with a fresh lancet. Then, the user opens a vial of strips, removes a strip and inserts a strip into the meter. The user then re-closes the vial and checks for the correct calibration code on the meter. Then the user picks up the lancing device and lances the skin of the finger or other body part. The user then lays down the lancing device and squeezes or massages the finger to yield an adequate blood sample. The user then transfers the sample to a test strip for analysis. Generally, a user is required to transfer a specific volume of sample to a specific location on the small test strip, a difficult task for many users. After transfer of the sample, the user waits for the meter to analyze the sample, then removes the strip from the test meter and discards the strip. Finally, the user re-packs all of the accessories. As set forth above, the standard regime for monitoring glucose requires the use of multiple, separate components.

U.S. Patent No. 5,871,494 describes a separate lancing apparatus for obtaining a sample of blood. The lancing apparatus can be connected to a glucometer for analysis of the blood sample.

WO 97/42882 describes an integrated sampling device for sampling bodily fluid and analyzing the fluid with a built-in analyzer.

The pain, inconvenience, cost, slowness, complexity and discreteness of running a blood test are barriers to the frequent monitoring of glucose levels. Patients often do not comply with doctor recommendations to frequently test glucose levels due to the numerous obstacles involved.

### SUMMARY OF THE INVENTION

In view of the foregoing and in accordance with one aspect of the present invention, there is provided an adapter for a testing device for testing analytes in bodily fluids comprising:
a housing;
a lancet assembly comprising a lancet drive train for holding and driving a lancet into the skin of a user;
a test port for receiving a test strip insertable into the test port; and
a housing connector for connecting the adapter housing to the testing device.

This way, a conventional testing device can be utilized and adapted with the adapter to provide an integrated testing device which is more convenient to use, thereby encouraging more frequent use of the testing device by a user.

The test strip is supplied to the adapter from a container separate to the adapter. This has the advantage that the adapter, even when connected to the testing device, is not bulky and is easy to use.

In one embodiment of the present invention, the adapter further comprises:
a strip connector for establishing electrical contact between a test strip in the test port of the adapter and the testing device. Thus, strips held in the test port are electrically connected to the testing device.

Preferably, the adapter further comprises a removable lancet cap including a pressure ring to assist extraction of a sample of bodily fluid.

Preferably, the adapter further comprises a dedicated storage compartment. The dedicated storage compartment can store one or more additional lancets.

In one embodiment of the present invention, the adapter further comprises a cocking button for cocking the lancet drive train.

Preferably, the adapter further comprises a trigger button for actuating the lancet drive train.

Preferably, the adapter further comprises a depth adjustment button for adjusting the penetration depth of the lancet. Thus, the penetration depth suitable for obtaining a sample of bodily fluid from a particular individual can be optimized.

Preferably, the angular position of the depth adjustment button defines a cocked position and a final extended position of the lancet.

Preferably, the depth adjustment button includes a first slanted protrusion for defining the cocked position and a second slanted protrusion that is parallel to the first slanted protrusion for defining the final extended position.

Preferably, the first slanted protrusion contacts a stop on the lancet drive train to define the cocked position.

In one embodiment of the present invention, the adapter further comprises a cocking button for cocking the lancet drive train, wherein the second slanted protrusion contacts a fixed cocking rib on the cocking button.

Preferably, the cocking button includes a stop for the lancet drive train, such that the position of the cocking button defines the final extended position of the lancet.

Preferably, the testing device is a glucose meter and the analyte being tested is glucose in a sample of blood.

In accordance with a second aspect of the present invention, there is provided a method of providing an integrated testing device, comprising:
providing a testing device for testing analytes in bodily fluids;
providing an adapter containing a housing, a lancet assembly, a test port for receiving a test strip insertable into the test port, a strip connector for establishing electrical contact between a test strip in the test port of the adapter and the testing device, and a housing connector for connecting the adapter housing to the testing device; and
attaching said adapter to said testing device using the housing connector.

In an illustrative embodiment, the present invention provides a clip-on adapter device for a standard glucose meter to convert the meter into an integrated single-unit testing device. The adapter comprises a lancing device, a test strip port for holding a test strip in close proximity to a puncture site, a strip connector for providing an electrical connection between the test strip and the electronics in the glucose meter and a connector for connecting the adapter to the glucose meter. The adapter further includes an adapter cap, including a pressure ring for facilitating the expression of blood from a puncture site. The adapter includes a cocking mechanism for cocking the lancing device, a depth control mechanism for varying the penetration depth of the lancet and a trigger for actuating the lancet.

According to the illustrative embodiment, the depth control mechanism comprises an arc-shaped substrate and two ribs disposed on the interior of the substrate. The ribs define the starting position and the stopping position of the lancet. A first rib interfaces with a stop on the lancet barrel to define the starting position of the lancet when the lancet is cocked. The second rib interfaces with a cocking rib on the cocking button to define the position of a stop located on the cocking button corresponding to the extended position of the lancet. The depth control mechanism is rotatably mounted on the adapter housing such that a knob of the depth control mechanism protrudes from the housing. A user rotates the depth control mechanism about the longitudinal axis of the adapter to vary the position of the two rib interfaces, and thus vary the penetration depth of the lancet.

The adapter of the present invention achieves in situ testing by converting a standard glucose meter into an integrated blood sampling and testing device. The user can attach the adapter to the glucose meter to integrate the process of obtaining a blood sample and testing the components of the blood sample. The use of the clip-on adapter with a standard glucose meter eliminates the need to transfer a blood sample from a sampling site to a test strip and the need to then transfer the test strip to a separate glucose meter. The adapter combines the separate accessories involved in glucose monitoring into a single, easy-to-use and convenient device. The adapter can also be detached from the glucose meter and used separately, if so desired.

### BRIEF DESCRIPTION OF THE DRAWINGS

These and other features and advantages of the present invention will be more fully understood by reference to the following detailed description in conjunction with the attached drawings in which like reference numerals refer to like elements through the different views.

A specific embodiment is now described by way of example only and with reference to the accompanying drawings, in which:
Figure 1 is a general view of the adapter of the illustrative embodiment of the invention attached to a standard glucose meter;
Figure 2 is a side view of the adapter and glucose meter assembly of Figure 1;
Figure 3 is a general view of the adapter and glucose meter assembly of Figure 1;
Figure 4 illustrates the adapter wherein a half of the housing and the cap are removed to expose the interior of the adapter;
Figure 5 is an exploded view of the lancing mechanism in the device;
Figure 6 illustrates the lancet barrel of the adapter;
Figures 7a and 7b illustrative the details of the depth adjustment button;
Figure 8 illustrates the lancing mechanism in a cocked position the cocking button removed for clarity;
Figures 9a and 9b illustrate the details of the cocking button of the adapter;
Figure 10 illustrates the assembled lancing mechanism during a cocking process;
Figure 11 is a general partial view of the adapter, illustrating the connector for connecting the adapter to a glucose meter; and
Figures 12a, 12b, 12c and 12d illustrate the strip contact extension feature for extending electrical contact from a test strip in the adapter to the glucose meter electronics.

### DETAILED DESCRIPTION OF THE ILLUSTRATIVE EMBODIMENT

The present invention is described below relative to an illustrative embodiment. Those skilled in the art will appreciate that the present invention may be implemented in a number of different applications and embodiments and is not specifically limited in its application to the particular embodiment depicted herein. The present invention is discussed below in connection with sampling blood for analysis by a glucose meter, although those of ordinary skill will recognize that other types of testing devices could be used for sampling and testing other fluids.

There is now described an illustrative embodiment of an adapter according to the present invention. The adapter is for use with a glucose meter to provide an integrated device for obtaining and analyzing a sample of bodily fluid, such as blood. The adapter facilitates the monitoring of blood glucose levels by providing a means for integrating into a single device the steps involved in sampling and analyzing blood. The integration results in a simplified process employing a single device.

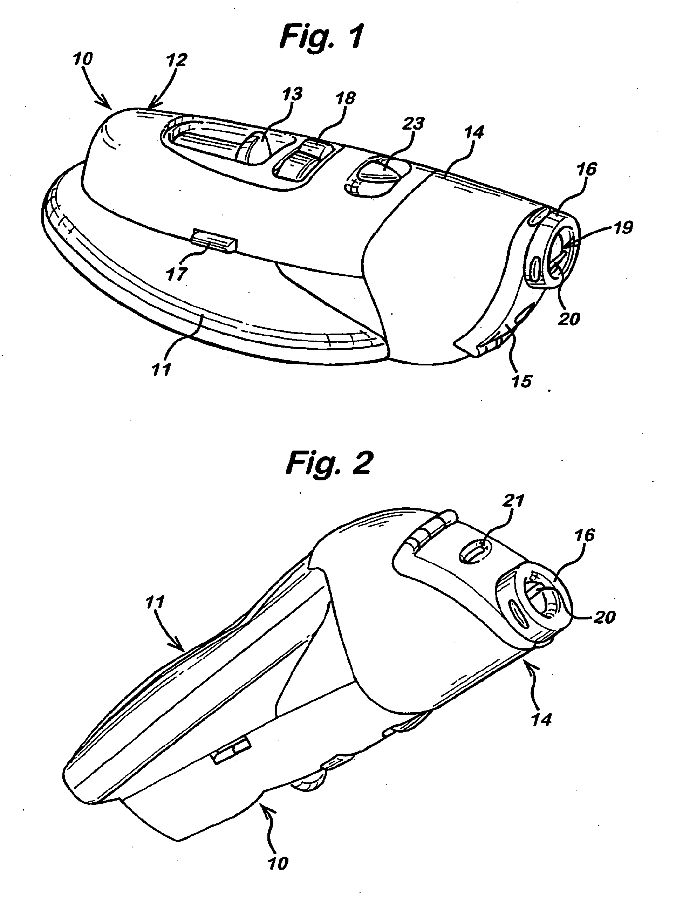

Figure 1 illustrates a clip-on adapter device 10 of the illustrative embodiment of the present invention. The adapter device 10 is attached to a standard glucose meter 11 to convert the glucose meter into an integrated device for sampling and testing a blood sample. The adapter device 10 includes a housing 12, a lancing mechanism (not shown), including a lancet for puncturing the skin of a user to express a drop of blood, a cocking button 13 for cocking the lancing mechanism and a depth adjustment button 18 for adjusting the penetration depth of the lancet. As shown, the cocking button 13 is slidably mounted in the housing and protrudes through longitudinal slot in the housing. According to the illustrative embodiment, a user retracts the cocking button towards the distal end of the housing to cock the lancing mechanism. The depth adjustment button 18 is rotatably mounted in the housing and protrudes through a second longitudinal slot in the housing. The user rotates the depth adjustment button 18 about its axis to vary the penetration depth of the lancet. After the user cocks the lancing mechanism and selects a penetration depth, the user depresses a triggering button 23 to release the lancet from the cocked position. A housing connector 17 connects the adapter 10 to the glucose meter 11 to form an integrated sampling and testing device. According to the illustrative embodiment, the housing connector 17 replaces the original battery door of the glucose meter and has the dual function of enclosing a battery compartment in the glucose meter and providing a connection to the adapter 10.

A removable lancet cap 14 is attached to the proximal end of the housing and includes an aperture 19 to allow passage of the lancet through the cap and into the skin of the user. According to the illustrative embodiment, the cap is substantially clear or includes a substantially clear portion to allow a user to view a sample being expressed from the puncture site when the lancet punctures the skin of the user. The cap 14 includes a pressure ring 16 disposed about the aperture 19. The pressure ring 16 has a multi-contoured surface in order to promote, enhance or facilitate the expression of blood by pressing the device onto the skin. A hinged door 15 provides access to the interior of the cap 14. According to the illustrative embodiment, a storage compartment (not shown) is provided within the interior of the cap 14, which allows a user to store spare lancets, pressure rings, test strips and the like.

The cap 14 for the adapter includes a pressure ring 16 attached to the distal end of the cap body. As discussed, the pressure ring 16 is mounted around and encloses the aperture 19 through which the lancet passes. The pressure ring 16 has a multi-contoured surface oriented generally about an axis distinct from the axis of motion of the lancet. The multi-contoured surface is designed to exert pressure on the dermal tissue to facilitate expression of a fluid sample after lancing the dermal tissue. According to an alternate embodiment, the pressure ring 16 comprises a pair of pressure wings sized and dimensioned to accommodate the sharp curve of the fingertip therebetween. The pressure wings thus form a recess for accommodating the finger of the user. This applies the correct amount of pressure to allow for the expression of blood.

A test strip 20 is positioned in an aperture 19 or a test port (not shown) and extends through the aperture 19. When the lancet punctures the skin and a drop of blood forms on the skin surface, the position of the test port locates the test strip in close proximity to receive the blood sample. The adapter further includes an extension (not shown) to the test port of the glucose meter 11, which extends the electrical contacts from the test port of the glucose meter to the contacts on the test strip 20. In this manner, in situ testing of the blood sample is enabled. The individual features and components of the adapter device 10 will be described in more detail below.

The adapter device 10 of the illustrative embodiment is configured to clip onto a standard glucose meter, such as a One Touch Ultra meter from Lifescan, the QID meter from Abbott, the Glucometer Elite meter from Bayer, the Advantage meter from Roche, and other commercially available glucose meters. The housing connector 17, which enables the attachable clip-on feature, may be customized for any suitable particular glucose meter employed by the user.

Figure 2 illustrates a side view of the adapter device 10 connected to the glucose meter 11 and including an alternative access port 21. As illustrated, the alternative access port 21 is provided in the hinged door 15. The alternative access port 21 accepts an alternative test strip such that contacts on the alternative test strip connect to the electrical contacts from the test strip port. The alternative access port 21 does not have a surrounding pressure ring 16 or lancet adjacent the alternative access port 21. This way, a test strip can be placed in the alternative access port 21, the skin of a user can be pricked with an independent lancing device to obtain a blood sample, and the blood sample can be applied to the test strip for analysis by the glucose meter 11.

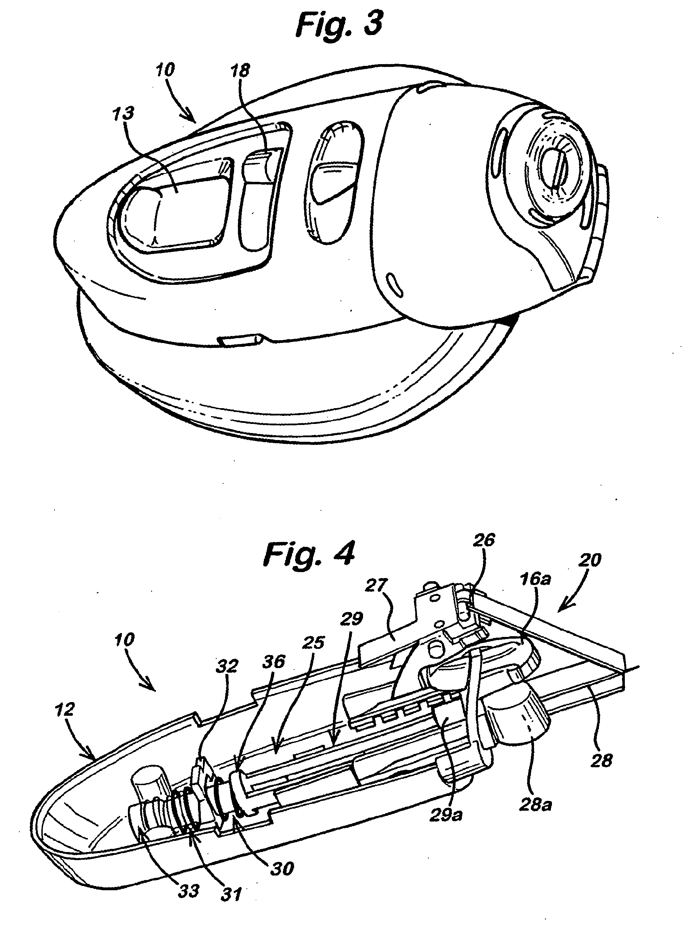

Figure 3 is a general view of the integrated device comprising the adapter 10 connected to the glucose meter 11. In Figure 3, the cocking button 13 is shown in a retracted position, corresponding to the end of a cocking process. The depth adjustment button 18 shown in Figure 3 is rotated to select a minimum puncture depth. As discussed, the puncture depth is determined by the position of the depth adjustment button, and the puncture depth is adjusted by rotating the button between a variety of positions. According to the illustrative embodiment, the depth adjustment button is rotated to the left side of the longitudinal slot to select a minimum depth and the right side of the longitudinal slot to select a maximum penetration depth. One skilled in the art will recognize that the depth adjustment feature is not limited to the illustrative embodiment. For example, the depth adjustment button 18 may be configured such that rotation to the right will decrease the penetration depth. Alternatively, the depth adjustment button may be configured such that a linear movement in a predetermined direction alters the penetration depth of the lancet.

Figure 4 illustrates the adapter 10 wherein a half of the housing 12 and the cap 14 are removed to expose the interior of the adapter 10. The interior of the adapter houses the lancing mechanism 25, a test strip 20, a set of electrical contact bars 26 and a printed circuit board 27 for connecting the test strip to the glucose meter. The lancing mechanism is slidably mounted within the housing of the adapter 10 and includes a disposable lancet 28 and a lancet drive train for driving the lancet into and out of the skin of a user. The lancet drive train comprises a lancet barrel 29 having a barrel collar 36 and a barrel base 33, a first spring 30, a second spring 31 and a spring retainer 32. The lancet 28 is releasably mounted in the proximal end 29a of the lancet barrel 29. The lancet barrel is connected to the cocking button (not shown in Figure 4), such that retraction of the cocking button towards the distal end of the adapter will retract the lancet barrel to a cocked position. In addition, Figure 4 illustrates the implementation of storage compartments within the housing of the adapter 10 for storing a spare lancet 28a and a spare or alternate pressure ring 16a.

The first spring 30 comprises a relatively strong spring for driving the lancet. The first spring 30 is housed between the barrel collar 36 and the spring retainer 32 and is compressed when the lancing mechanism is retracted to the cocked position. According to the illustrative embodiment, the amount of compression in the first spring 30 when the lancet is fully cocked depends on the position of the depth selection button, which will be described in detail below. The first spring 30 has a restoring force that is proportional to the amount of compression. When the lancet barrel is released from the cocked position by depressing the trigger button, the restoring force propels the lancet along a predetermined path through the aperture 19 of the adapter. The second spring 31 comprises a relatively weak spring and is housed between an interior rib of the cocking button (not shown in Figure 4) and the barrel base 33. The weak spring 31 is also compressed as the cocking button is retracted to cock the lancet. When the user releases the cocking button, the weak spring biases the cocking button back to a rest position, while the lancet barrel remains in a locked cocked position.

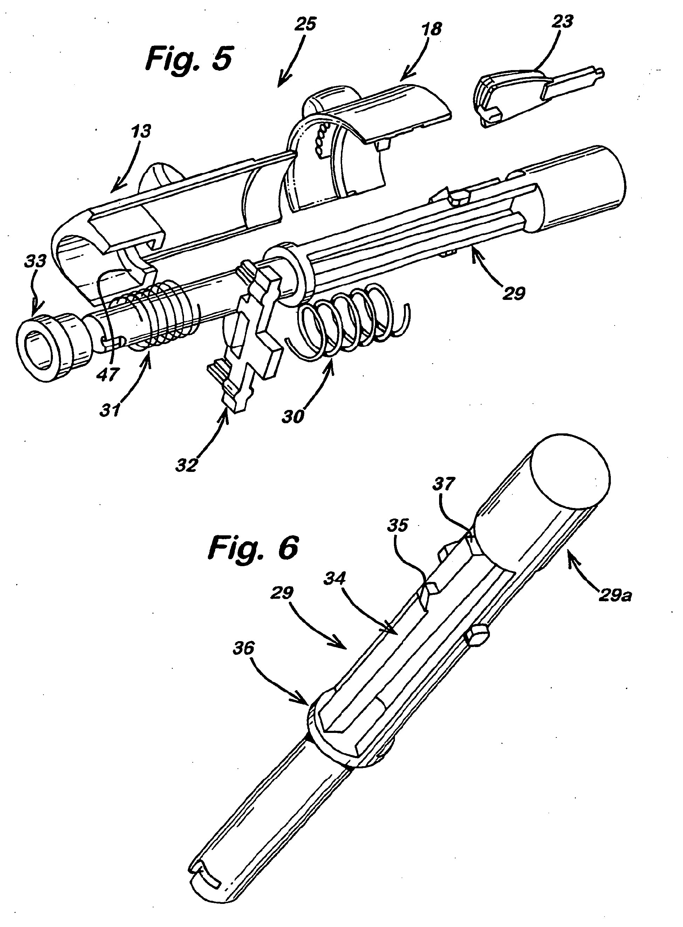

Figure 5 is an exploded view of the lancing mechanism 25, illustrating the different components and the relative positions of the respective components involved in the lancing and depth adjustment functions. The trigger button 23, depth adjustment button 18 and the cocking button 13 are arranged relative to each other and the lancet barrel 29 and cooperate to arm the lancing mechanism, fire the lancing mechanism and control the penetration depth of the lancet. As discussed, the cocking button 13 operates to retract the lancet barrel 29 from a rest position to a cocked position. The barrel is temporarily locked in the cocked position by a rib 41 located on the interior side of the depth adjustment button 18. The position of the depth adjustment button 18 determines the starting position (i.e. the cocked position) of the lancet 28 before the lancet is released and penetrates the skin. The trigger button 23 releases the barrel 29 from the cocked position when a user depresses the trigger button. Upon release, the lancet travels a predetermined distance until the barrel base 33 abuts an internal rib 47 of the cocking button, which stops the lancet travel. The first spring 30 then retracts the lancet from the skin. The working and functionality of the different components, particularly the lancet barrel 29, the cocking button 13, the depth adjustment button 18 and the trigger button 23 will be described in detail below.

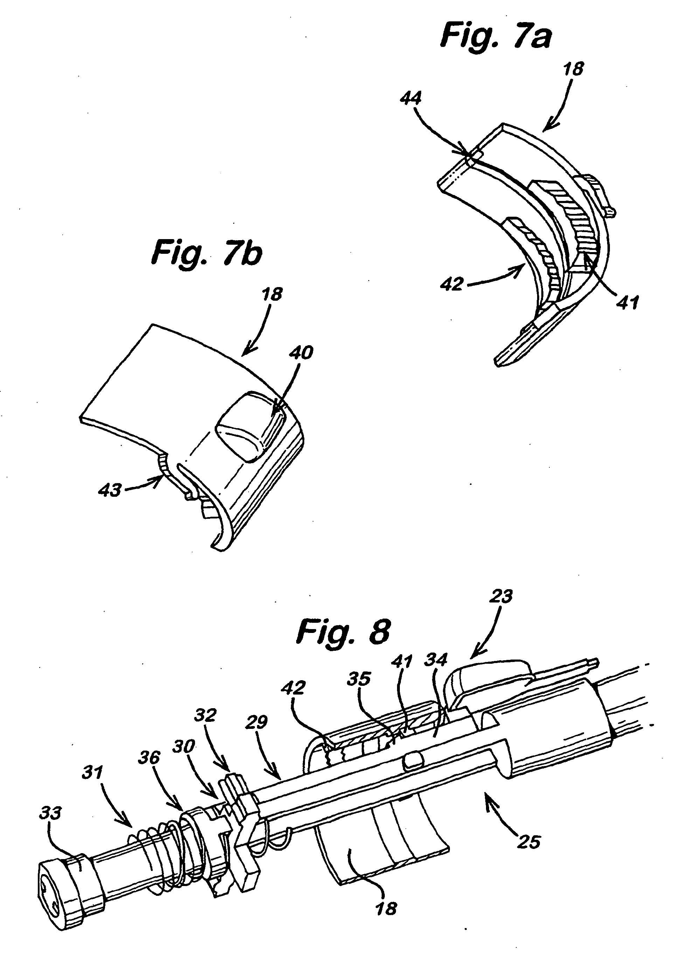

Figure 6 illustrates the lancet barrel 29 of the adapter 10 according to the illustrative embodiment. The proximal end 29a of the lancet barrel is configured to hold a lancet 28. The barrel 29 includes a flexible arm 34 and a catch 35 on the flexible arm for temporarily locking the barrel 29 in a cocked position. According to the illustrative embodiment, the catch 35 interfaces with a rib 41 on the depth adjustment button, shown in Figure 7 and described in detail below, to lock the barrel in the cocked position. According to the illustrative embodiment, the position of the cocked lancing mechanism is determined by the angular position of the depth adjustment button 18. The trigger button 23 cooperates with an end portion 37 of the flexible arm 34 to release the barrel 29 from the cocked position. When a user depressed the trigger button 23, the trigger presses the end portion 37, which forces the flexible arm 34 to flex, thereby releasing the catch 35 from the rib of the depth adjustment button 18.

Figures 7a and 7b illustrate the depth adjustment button 18 according to the illustrative embodiment. The depth adjustment button 18 allows a user to select a preferred penetration depth and customize the adapter to different people and different body parts. The depth adjustment button 18 comprises an arc-shaped substrate that is rotatably mounted in the housing 12 of the adapter and extends through a longitudinal slot in the housing 12, as illustrated in Figures 1-3. A knob 40 disposed on the exterior side of the depth adjustment button 18 may be grasped by a user to rotate the depth adjustment button about the longitudinal axis of the adapter 10 to select the desired penetration depth. A rim 44 on the depth adjustment button 18 cooperates with a groove in the housing to facilitate rotational movement of the depth adjustment button. The angular position of the depth adjustment button 18 determines the depth of the lancing mechanism. To increase the penetration depth, a user rotates the depth adjustment button in a first direction. To decrease the penetration depth, the user rotates the depth adjustment button in an opposite direction.

To provide the variable depth feature, a first rib 41 and a second rib 42 are disposed on an interior side of the depth adjustment button 18. According to the illustrative embodiment, the ribs 41 and 42 are slanted to allow selection of variable penetration depths, to provide smooth transition between different positions, and to accommodate the limited space underneath the surface of the depth adjustment button 18. The first rib 41 cooperates with the catch 35 on the flexible arm of the lancet barrel to define a cocked position of the lancet, as described above. As shown, the longitudinal position of any segment of the first rib 41 is controlled by the rotation of the button around its axis. The second rib 42 is parallel to the first rib and interfaces with a cocking rib 46 on the cocking button 13, to be described in detail below, to define the final penetration depth of the lancet. The cocking rib 46 on the cocking button 13 abuts the second rib 42 to define the rest position of the cocking button 13. The cocking button in turn defines a stop for the lancet mechanism, thereby controlling the penetration depth of the lancet 28. (The position of the depth adjustment button defines the position of the cocking button, which defines the stop position of the lancet). The rotation of the depth adjustment button 18 brings different portions of the ribs 41, 42 into the path of the catch of the lancet barrel 29 and the cocking rib of the cocking button 13, respectively. When rotated to the left, the rib interfaces (i.e. the starting and stopping positions of the lancet) are moved back towards the distal end of the adapter, decreasing the amount that the lancet protrudes from the aperture. When rotated to the right, the rib interfaces move forwards, towards the proximal end of the adapter, thereby increasing the amount that the lancet protrudes from the aperture. A depth selection arm 43 allows a user to select a discrete depth position when rotating the depth adjustment button 18 about its axis. The arm 43 "clicks" into each discrete depth position to indicate to the user that a particular depth has been selected. The depth adjustment feature provides a simplified and accurate means for ensuring that a desired penetration depth is achieved. One skilled in the art will recognize that the depth adjustment button is not limited to the illustrative configuration and that any suitable shape or arrangement may be utilized to select a penetration depth for a lancet and that the present invention is not limited to the illustrative embodiment.

Figure 8 illustrates the lancing mechanism in a cocked position. As shown, the depth adjustment button 18 holds the lancing mechanism in the cocked position. As the lancing mechanism, including the barrel 29, is retracted using the cocking button, the catch 35 of the barrel 29 is caught behind the first rib 41 of the depth adjustment button to temporarily lock the barrel in the cocked position. As shown, the position of the depth adjustment button 18 defines the starting position of the lancet, and rotation of the depth adjustment button varies the starting position of the lancet. In Figure 8, the depth adjustment button 18 is rotated to the left to move the starting position back towards the distal end of the adapter and thereby produce a shorter penetration depth. As discussed, the trigger 23 is depressed to flex the flexible arm 34 and release the lancet barrel catch from behind the first rib 41.

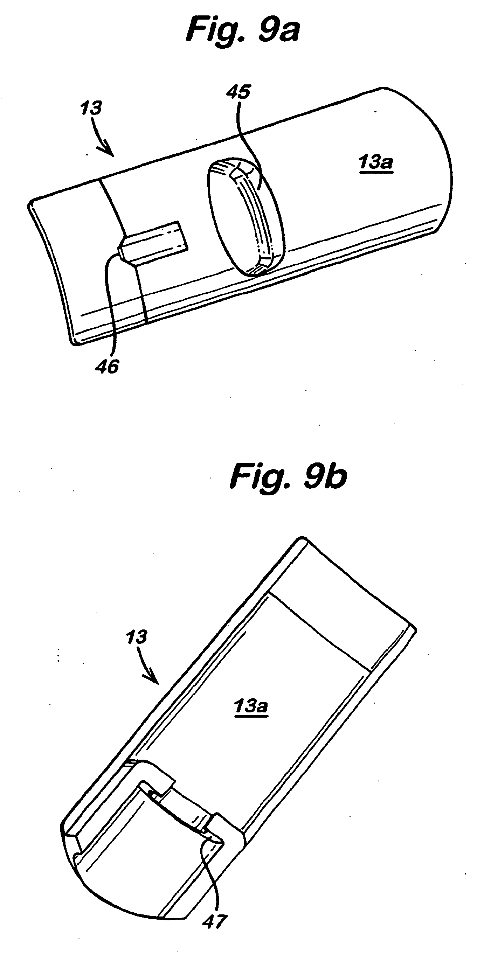

Figures 9a and 9b illustrate the cocking button 13 of the illustrative embodiment. As discussed, the cocking button 13 is slidably mounted in the housing 12 of the adapter 10 of the illustrative embodiment and extends through a longitudinal slot in the housing. The cocking button 13 of the illustrative embodiment comprises a substrate 13a, a cocking knob 45 and a cocking rib 46 disposed on the exterior surface of the substrate 13a and an interior rib 47 disposed on the interior surface of the substrate 13a. The cocking button 13 is connected to the lancing mechanism 25 and adapted to slide in the longitudinal slot.

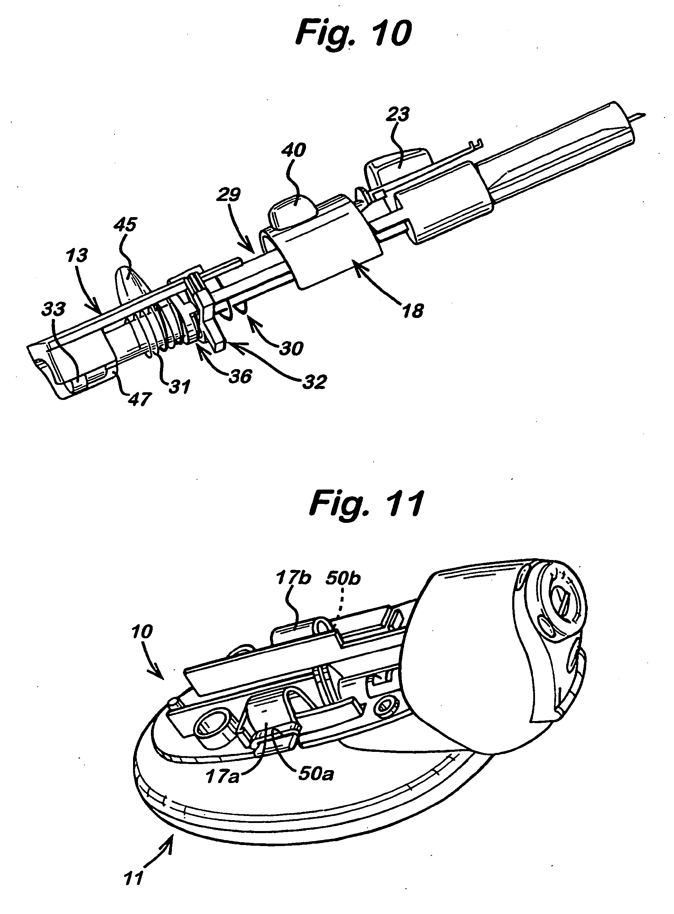

Figure 10 illustrates the assembled lancing mechanism, including the cocking button, the trigger button and the depth adjustment button during the cocking process. To cock the lancing mechanism, a user grasps the cocking knob 45 and slides the cocking button 13 towards the distal end of the adapter 10. The interior rib 47 on the cocking button 13 abuts the barrel base 33 and forces the barrel base 33 to retract with the cocking button. During the cocking process, the weak spring 31 becomes compressed between the interior rib 47 and the barrel base 33. The strong spring 30 becomes compressed between the barrel collar 36 and the spring retainer 32. The first rib of the depth adjustment button 18 captures the catch 35 of the lancet barrel 29 to hold the lancing mechanism in the cocked position, such that the strong spring remains compressed 30. The weak spring 31 of the lancing mechanism biases the cocking button back towards the proximal end of the adapter, until the cocking rib 46 on the exterior surface of the cocking button 13 abuts the second rib on the interior of the depth adjustment button 18. The interface between the cocking rib 46 and the cocking button 13 defines the rest position of the cocking button 13. The interior rib 47 of the cocking button includes an opening sized and dimensioned to allow the lancet barrel 29 to slide through while retaining the barrel base 33. The interior rib 47 serves as a stop for the lancet. When the drive train drives the lancet 28 through the aperture 19 in the adapter cap 14, the interior rib 47 stops lancet at a predetermined travel depth. As discussed, the position of the interior rib 47, and thus the penetration depth of the lancet, is defined by the position where the cocking rib 46 strikes the second slanted rib 42 of the depth adjustment button 18, which is in turn defined by the axial position of the depth adjustment button 18.

The lancing mechanism of the illustrative embodiment, including the system and method for adjusting the penetration depth, may be used alone, and is not limited to use in an adapter for a glucose meter, as illustrated. According to an alternate embodiment of the invention, the illustrated lancing mechanism may be utilized in a stand-alone lancet device that is not adapted to attach to a glucose meter, which incorporates the depth adjustment capabilities described herein.

Figure 11 is a general partial view of the adapter 10 of the present invention connected to a glucose meter 11. Figure 11 illustrates the attachment of the adapter 10 to the glucose meter using a housing connector 17 specially designed to integrate the adapter device with the meter. According to the illustrative embodiment, the housing connector 17 is configured to replace the original battery door of the meter. The housing connector 17 replicates the battery door and covers the batteries (not shown) in the glucose meter in the same way. The housing connector 17 further includes protrusions 17a and 17b, which are configured to secure the adapter 10 to the glucose meter 11. As shown, the adapter 10 includes slots 50a, 50b for receiving the protrusions 17a, 17b. One skilled in the art will recognize that any suitable means for releasably attaching the adapter device 10 to a glucose meter may be utilized.

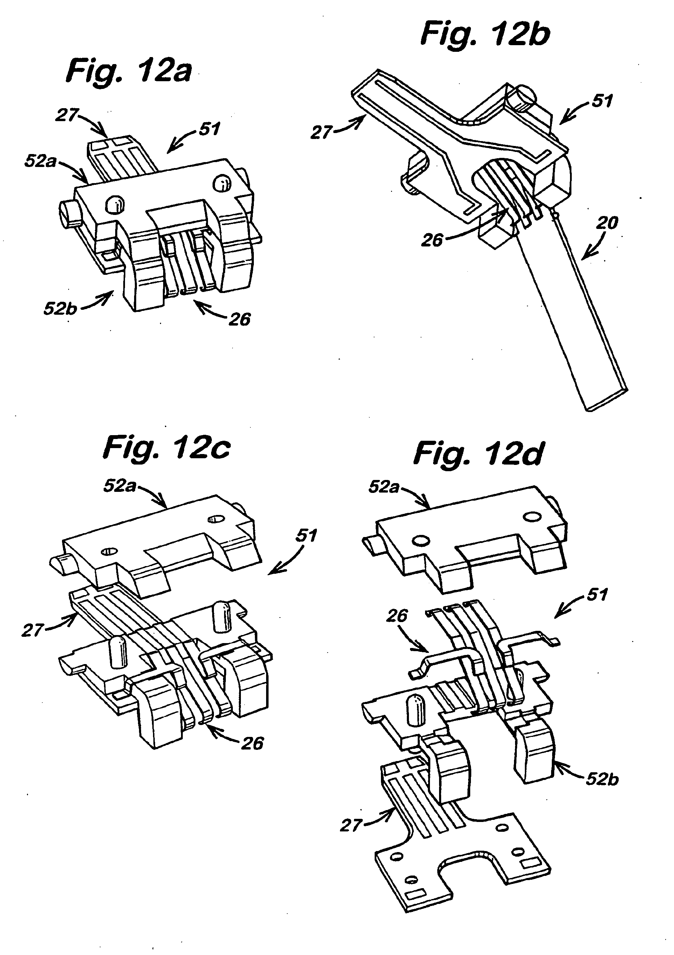

The adapter further includes a strip contact extension for facilitating in situ electrochemical analysis of the sample by connecting the test strip inserted into a test port to the attached glucose meter. The strip contact extension feature is illustrated in detail in Figures 12a, 12b, 12c and 12d. The strip connector 51 establishes electrical contact between the original test port of the glucose meter and the test strip 20 in the adapter. The electrical contact bars 26 on the strip connector contact electrodes formed on the test strip 20. The electrical contacts are connected to a printed circuit board 27 which contacts the original contacts (not shown) of the glucose meter. A substrate, comprising two parts 52a and 52b are used to assemble the electrical contact bars 26 and printed circuit board 27. The strip connector 51 is connected to the adapter such that the printed circuit board will contact the original contacts of the glucose meter when the adapter is attached to the glucose meter. The original contacts of the glucose meter connect to electronics located within the glucose meter housing. The test strip generates electrochemical signals that are passed by the strip connector to the glucose meter electronics. As known in the art, the electronics in the glucose meter process the signal and calculate the glucose level or other electrochemically detectable analyte of the blood or other interstitial fluid that is sampled by the testing device. The electronics transmit instructions for an appropriate display or output regarding the analysis.

The test strip 20 essentially comprises an electrochemical cell, including one or more working electrodes, which convert a chemical change produced by a reaction of glucose or other analyte in the blood sample to a current. The test strip 20 further includes a reference electrode as a standard to measure the potential of the working electrodes. Leads connect the electrodes to the contact bars 26 of the strip connector 51, which establishes electrical contact with the electronics of the glucose meter. The test strip thus generates a signal indicative of the level of glucose or other analyte in the blood and transmits this signal to the electronics of the device for processing. Those skilled in the art will recognize that a variety of test strip designs and configurations are available in accordance with the teachings of the present invention.

The illustrative embodiment of the invention achieves in situ testing of a blood sample based on proximity to a lancet wound. When the lancet 28 punctures the skin, a drop of blood forms on the skin surface. The test strip 20 is moved into close proximity to the puncture wound to ensure that blood contacts the strip. Moreover, the positioning of the test strip adjacent the lancet ensures that only small volumes of blood are required. Preferably the test strip is separated from or positioned relative to the deployed lancet or lancing site between about 0.4 mm and about 1.3 mm, and preferably between about 0.7 mm and about 0.9 mm. Once the drop grows to a certain size, the drop touches the entrance of the capillary channel and is consequently drawn into the strip for analysis. The test strip wicks the blood sample directly from the puncture wound, allowing low volume blood samples for analysis. The test strip is positioned to automatically and efficiently direct the sample to a defined area of the test strip for analysis. The described arrangement efficiently conveys a sample from the skin to a precise position on the test strip with little to no losses. Furthermore, the established electrical connection between the test strip 20 and the glucose meter electronics, provided by the strip connector 51 of the adapter 10, allows analysis of the sample to occur without necessitating transfer of the sample or the test strip.

To measure blood glucose levels, a user first attaches the adapter 10 to the glucose meter 11 to provide an integrated sampling and testing device. The user inserts at test strip 20 into the test port, such that the test strip is in close proximity to the puncture site. The user cocks the lancing drive train by pulling back the cocking button 13, and subsequently releases the cocking button, which automatically returns to a rest position. The user selects a suitable penetration depth by rotating the depth adjustment button 18, the angular position of which defines the starting and stopping position of the lancet 28. The user then presses the adapter against a body part, such as a finger, such that the skin of the user contacts the pressure tip 16. The user depresses the trigger button 23 to release the lock on the lancet barrel 29 formed by the interface between the first rib 41 of the depth adjustment button 18 and the flexible arm 34 of the barrel. Consequently, the restoring force in the strong spring 30 drives the lancet tip 28 a predetermined depth into the skin, in close proximity to the test strip 20. The barrel base 33 abuts the interior rib 47 of the cocking button 13 to stop the lancet at the predetermined depth. The lancet assembly immediately retracts the lancet from the skin, yielding a drop of blood or other bodily fluid on the skin surface. According to one practice of the invention, the pressure tip 16 in the adapter cap 14 squeezes the skin to maximize the quantity of blood formed from a puncture. As the blood drop grows, the blood contacts a channel entry of the proximally located test strip 20, which absorbs the blood sample and directs the blood sample to an analysis portion. The analysis portion of the test strip generates a signal indicative of glucose levels, which is automatically provided to the glucose meter via the strip connector 51. In situ testing of the blood sample is enabled by virtue of the location of the adapter and test strip. In other words, the adapter allows the blood sample to be tested without necessitating manual transfer of the sample from the puncture site to the glucose meter. The glucose meter 11 displays and stores the glucose reading. After the analysis is complete, the user removes the adapter cap 14 and the lancet 28 from the lancet barrel 29. The user may then discard the lancet, if desired.

The illustrative embodiment of the invention provides significant advantages to testing and sampling blood by providing a device for integrating the sampling and testing of blood into a single, user-friendly device. The adapter can be retrofitted to an existing glucose meter to convert the glucose meter to have sampling capabilities. The adapter can also be detached and used separately from the glucose meter, if desired. The adapter encourages frequent use by reducing the pain, inconvenience and complexity of performing a blood test. The adapter reduces the number of accessories and steps by integrating all of the different accessories involved in glucose monitoring into one unit. The dedicated storage space provided in the adapter cap allows a user to store spare lancets, test strips and pressure tips conveniently and within easy reach. The invention further improves the efficiency and accuracy of testing by providing an automated transfer and analysis of the sample. The use of the clip-on adapter with a standard glucose meter eliminates the need to transfer a blood sample from a sampling site to a test strip and the need to then transfer the test strip to a separate glucose meter. The integrated testing device enabled by the adapter is compact, ergonomically sound, discrete and adjustable to different users and body parts while simultaneously providing fast and accurate results.

The present invention achieves pain-free testing in a number of ways. Shallower penetrations of the skin can be used to achieve a sufficient blood sample, reducing painful deep incisions in sensitive body parts. The present invention requires low sample volumes for analysis. The finger squeezing mechanism formed by the pressure tip on the lancet cap provides a high yield for small puncture wounds. The integrated sampling and testing feature further ensures full usage of the obtained sample and limits "leftovers" on the skin. In current systems, complex and inaccurate sample transfer from a sampling point to an application area on a test strip requires surplus sample due to poor utilization of an obtained sample drop. The present invention removes the inefficiency of transferring samples and provides optimal utilization of the obtained sample by automatically directing the sample to a precise location on the test strip. The superficial penetrations reduce agitation of nerve endings in the skin and reduce pain in sensitive body areas.

The variable depth of the lancet and the ability to test on a number of different body parts in addition to the finger reduces the concentration and repetition of micro-traumata in a small area, which result in tinting, itching, dried and callous skin areas. The illustrated depth adjustment mechanism, whether implemented in the adapter device or in a standard lancing device, provides a user-friendly means for precisely controlling the penetration depth of the lancet.

The present invention further provides easy and uncomplicated operation. The sampling and testing device significantly reduces the time and difficulty involved in sampling and testing blood. The adapter is designed such that one-handed operation is possible, eliminating the need for a separate workspace. The fully automated testing system is not subject to human error and inefficiency. The present invention also reduces waste by efficiently utilizing available resources. The present invention further protects against compromised test results due to contamination or an improperly calibrated glucose meter.

In conclusion, the integrated sampling and testing device of the present invention significantly reduces the obstacles associated with frequent glucose monitoring. The present invention promotes frequent monitoring for diabetic individuals by providing a simple, efficient, fast and accurate integrated sampling and testing device.

Since certain changes may be made in the above constructions without departing from the scope of the invention, it is intended that all matter contained in the above description or shown in the accompanying drawings be interpreted as illustrative and not in a limiting sense.

In addition, it will of course be understood that the present invention has been described above purely by way of example and modifications of detail can be made within the scope of the invention.

## Claims

1. An adapter for a testing device for testing analytes in bodily fluids comprising:
a housing;
a lancet assembly comprising a lancet drive train for holding and driving a lancet into the skin of a user;
a test port for receiving a test strip insertable into the test port;
a housing connector for connecting the adapter housing to the testing device; and
a strip connector for establishing electrical contact between a test strip in the test port of the adapter and the testing device.

2. The adapter of claim 1, further comprising a removable lancet cap including a pressure ring to assist extraction of the blood sample.

3. The adapter of claim 2, further comprising a dedicated storage compartment.

4. The adapter of any one of claims 1 to 3, further comprising a cocking button for cocking the lancet drive train.

5. The adapter of any one of claims 1 to 4, further comprising a trigger button for actuating the lancet drive train.

6. The adapter of any one of claims 1 to 5, further comprising a depth adjustment button for adjusting a penetration depth of the lancet.

7. The adapter of claim 6, wherein the angular position of the depth adjustment button defines a cocked position and a final extended position of the lancet.

8. The adapter of claim 7, wherein the depth adjustment button includes a first slanted protrusion for defining the cocked position and a second slanted protrusion that is parallel to the first slanted protrusion for defining the final extended position.

9. The adapter of claim 8, wherein the first slanted protrusion contacts a stop on the lancet drive train to define a cocked position.

10. The adapter of claim 8 or claim 9, further comprising a cocking button for cocking the lancet drive train, wherein the second slanted protrusion contacts a fixed cocking rib on the cocking button.

11. The adapter of claim 10, wherein the cocking button includes a stop for the lancet drive train, such that the position of the cocking button defines the final extended position of the lancet.

12. The adapter ofany one of the preceding claims, wherein the testing device is a glucose meter and the analyte being tested is glucose in a sample of blood.

13. A method of providing an integrated testing device, comprising:
providing a testing device for testing analytes in bodily fluids;
providing an adapter containing a housing, a lancet assembly, a test port for holding a test strip, a strip connector for establishing electrical contact between a test strip in the test port of the adapter and the testing device, and a housing connector for connecting the adapter housing to the testing device;
inserting a test strip into the test port of the adapter; and
attaching said adapter to said testing device using the housing connector.

## Patentansprüche

1. Adapter für ein Testgerät zum Prüfen von Analyten in Körperfluiden, mit:
einem Gehäuse;
einer Lanzenanordnung, die einen Lanzenantrieb zum Halten und Treiben einer Lanze in die Haut eines Benutzers aufweist;
einem Testport zum Aufnehmen eines Teststreifens, der in den Testport einsetzbar ist;
einem Gehäuseverbinder zum Verbinden des Adaptergehäuses mit dem Testgerät; und
einem Streifenverbinder zum Einrichten eines elektrischen Kontaktes zwischen einem Teststreifen in dem Testport des Adapters und dem Testgerät.

2. Adapter nach Anspruch 1, weiter mit einer entfernbaren Lanzenkappe, die einen Druckring umfaßt, um beim Herausziehen der Blutprobe zu unterstützen.

3. Adapter nach Anspruch 2, weiter mit einem zweckbestimmten Speicherabteil.

4. Adapter nach einem der Ansprüche 1 bis 3, weiter mit einem Spannknopf zum Spannen des Lanzenantriebs.

5. Adapter nach einem der Ansprüche 1 bis 4, weiter mit einem Auslöseknopf zum Betätigen des Lanzenantriebs.

6. Adapter nach einem der Ansprüche 1 bis 5, weiter mit einem Tiefeneinstellschalter zum Einstellen einer Eindringtiefe der Lanze.

7. Adapter nach Anspruch 6, bei dem die Winkelposition des Tiefeneinstellschalters eine Spannposition und eine schließlich ausgefahrene Position der Lanze definiert.

8. Adapter nach Anspruch 7, bei dem der Tiefeneinstellschalter einen ersten abgeschrägten Vorsprung zum Definieren der Spannposition und einen zweiten abgeschrägten Vorsprung, der parallel zu dem ersten abgeschrägten Vorsprung ist, zum Definieren der schließlich ausgefahrenen Position umfaßt.

9. Adapter nach Anspruch 8, bei dem der erste abgeschrägte Vorsprung einen Anschlag auf dem Lanzenantrieb berührt, um eine Spannposition zu definieren.

10. Adapter nach Anspruch 8 oder Anspruch 9, weiter mit einem Spannknopf zum Spannen des Lanzenantriebs, wobei der zweite abgeschrägte Vorsprung eine feste Spannrippe auf dem Spannknopf berührt.

11. Adapter nach Anspruch 10, bei dem der Spannknopf einen Anschlag für den Lanzenantrieb umfaßt, so daß die Position des Spannknopfes die schließlich ausgefahrene Position der Lanze definiert.

12. Adapter nach einem der vorangehenden Ansprüche, bei dem das Testgerät ein Glukosemeßgerät ist und der Analyt, der geprüft wird, Glukose in einer Blutprobe ist.

13. Verfahren zum Bereitstellen eines integrierten Testgerätes, das aufweist:
Bereitstellen eines Testgerätes zum Prüfen von Analyten in Körperfluiden;
Bereitstellen eines Adapters, der ein Gehäuse, eine Lanzenanordnung, einen Testport zum Halten eines Teststreifens, einen Streifenverbinder zum Einrichten eines elektrischen Kontaktes zwischen einem Testreifen in dem Testport des Adapters und dem Testgerät und einen Gehäuseverbinder zum Verbinden des Adaptergehäuses mit dem Testgerät enthält;
Einsetzen eines Teststreifens in den Testport des Adapters; und
Befestigen des Adapters an dem Testgerät, wobei der Gehäuseverbinder verwendet wird.

## Revendications

1. Adaptateur pour un dispositif d'essai pour tester des analytes dans des fluides corporels comprenant:
un boîtier;
un ensemble de lancette comprenant un mécanisme d'entraînement de lancette pour maintenir et entrainer une lancette dans la peau d'un utilisateur;
un orifice d'essai pour recevoir une bandelette d'essai insérable dans l'orifice d'essai; et
un connecteur de boîtier pour raccorder le boîtier d'adaptateur au dispositif d'essai; et un connecteur de bandelette pour établir un contact électrique entre une bandelette d'essai dans l'orifice d'essai de l'adaptateur et le dispositif d'essai.

2. Adaptateur selon la revendication 1, comprenant en outre un capuchon de lancette amovible comprenant un anneau de pression pour faciliter l'extraction de l'échantillon de sang.

3. Adaptateur selon la revendication 2, comprenant en outre un compartiment de stockage dédié.

4. Adaptateur selon l'une quelconque des revendications 1 à 3, comprenant en outre un bouton d'armement pour armer le mécanisme d'entraînement de lancette.

5. Adaptateur selon l'une quelconque des revendications 1 à 4, comprenant en outre un bouton déclencheur pour actionner le mécanisme d'entraînement de lancette.

6. Adaptateur selon l'une quelconque des revendications 1 à 5, comprenant en outre un bouton de réglage de profondeur pour ajuster la profondeur de pénétration de la lancette.

7. Adaptateur selon la revendication 6, dans lequel la position angulaire du bouton de réglage de profondeur définit une position armée et une position déployée finale de la lancette.

8. Adaptateur selon la revendication 7, dans lequel le bouton de réglage de profondeur comprend une première protubérance inclinée pour définir la position armée et une seconde protubérance inclinée qui est parallèle à la première protubérance inclinée pour définir la position déployée finale.

9. Adaptateur selon la revendication 8, dans lequel la première protubérance inclinée est en contact avec un arrêt sur le mécanisme d'entraînement de lancette pour définir une position armée.

10. Adaptateur selon la revendication 8 ou la revendication 9, comprenant en outre un bouton d'armement pour armer le mécanisme d'entraînement de lancette, dans lequel la seconde protubérance inclinée est en contact avec une nervure d'armement fixe sur le bouton d'armement.

11. Adaptateur selon la revendication 10, dans lequel le bouton d'armement comprend un arrêt pour le mécanisme d'entraînement de lancette, de sorte que la position du bouton d'armement définit la position déployée finale de la lancette.

12. Adaptateur selon l'une quelconque des revendications précédentes, dans lequel le dispositif d'essai est un glucomètre et l'analyte à déterminer est le glucose dans un échantillon de sang.

13. Procédé de production d'un dispositif d'essai intégré, comprenant les étapes consistant à:
produire un dispositif d'essai pour déterminer des analytes dans des fluides corporels;
produire un adaptateur contenant un boîtier, un ensemble de lancette, un orifice d'essai pour contenir une bandelette d'essai, un connecteur de bandelette pour établir un contact électrique entre une bandelette d'essai dans l'orifice d'essai de l'adaptateur et le dispositif d'essai, et un connecteur de boîtier pour connecter le boîtier d'adaptateur au dispositif d'essai;
insérer une bandelette d'essai dans l'orifice d'essai de l'adaptateur; et
fixer ledit adaptateur audit dispositif d'essai en utilisant le connecteur de boîtier.
